# EUROPEAN PATENT SPECIFICATION

(11) **EP 2 544 557 B1**
(45) Date of publication and mention of the grant of the patent: **21.02.2018**
(21) Application number: 11712032.9
(22) Date of filing: 11.03.2011
(51) Int. Cl.: A23L 1/29, G01N 33/50

(54) **NOVEL GLUCOSE TOLERANCE TEST AND COMPOSITION FOR USE**
NEUARTIGER GLUKOSETOLERANZTEST UND ZUSAMMENSETZUNG ZUR VERWENDUNG
NOUVEAU TEST DE TOLÉRANCE AU GLUCOSE ET COMPOSITION POUR SON UTILISATION

(30) Priority: 11.03.2010 WO PCT/NL2010/050128
(43) Date of publication of application: 16.01.2013
(73) Proprietor: N.V. Nutricia, 2712 HM Zoetermeer (NL)
(72) Inventor: HAGEMAN, Robert, Johan, Joseph, NL-6705 CT Wageningen (NL); LANSINK, Mirian, NL-3524 BJ Utrecht (NL); VAN HELVOORT, Adrianus, Lambertus, Bertholdus, NL-6703 ED Wageningen (NL); VAN NORREN, Klaske, NL-6871 LP Renkum (NL)
(74) Representative: V.O.
(86) International application number: PCT/NL2011/050172
(87) International publication number: WO 2011/112091

(56) References cited:
- EP-A1- 1 424 074
- WO-A1-2005/017532
- WO-A2-97/02050
- US-A1- 2005 038 329

## Description

### FIELD OF THE INVENTION

The present invention relates to a liquid nutritional composition, to a liquid nutritional composition for use in a diagnostic method for screening of a glucose-intolerant condition, to a kit for screening of a glucose-intolerant condition, and to a diagnostic method for screening a mammal for a glucose-intolerant condition. Furthermore, it refers to a liquid nutritional composition for use as a standard reference nutrition, such as for classification of postprandial glucose responses of different nutritional compositions.

### BACKGROUND OF THE INVENTION

Diabetes is a serious, lifelong disease which can cause long-term complications that affect almost every part of the body, leading to blindness, heart and blood vessel disease, strokes, kidney failure, amputations and nerve damage. It is widely recognized as one of the leading causes of death and disability in the US. Hence, an early and appropriate diagnosis is crucial for prevention and delaying the complications of diabetes. To this end, several tests, such as the oral glucose tolerance test (OGTT), have been devised, the latter test being at present the most common and frequently advised test for screening of glucose-intolerant conditions. Said oral glucose tolerance test in medical practice involves the administration of a bolus of pure synthetic glucose to determine the reaction of the body's metabolism on the glucose in the blood.

The OGTT test is usually used to test for diagnose diabetes, gestational diabetes (diabetes during pregnancy), pre-diabetes (a condition characterized by higher-than-normal blood sugar levels that can lead to type 2 diabetes), impaired glucose tolerance, impaired fasting glycaemia, insulin resistance, and sometimes reactive hypoglycaemia. The glucose bolus is most often given orally, so the common test is technically an oral glucose tolerance test (OGTT). The test may be performed as part of a panel of tests, such as the comprehensive metabolic panel. According to the National Institutes of Health (NIH), the OGTT is better able to diagnose high blood glucose after a glucose challenge than the fasting blood glucose test, which is a commonly used indication of overall glucose homeostasis. A medical practitioner may recommend it if the practitioner suspects a diabetic condition in cases where a subject's fasting blood glucose level is normal.

The OGTT is performed as follows. Before performing the OGTT test on a subject, the subject should have been fasting for the previous 8-14 hours (water is allowed). Usually the OGTT is scheduled to begin in the morning (between 7.00 and 8.00 AM) as glucose tolerance exhibits a diurnal rhythm with a significant decrease in the afternoon. A zero time (baseline) blood sample is drawn. The subject is then given a glucose solution to drink. The standard dose since is 1.75 grams of glucose per kilogram of body weight, to a maximum dose of 75 g. It should be drunk within 5 minutes. Blood is drawn at intervals for measurement of glucose (blood sugar), and sometimes insulin levels. The intervals and number of samples vary according to the purpose of the test. For normal screening for glucose-intolerant conditions, the most important sample is the 2 hour sample and the 0 and 2 hour samples may be the only ones collected. In particular, if renal glycosuria (sugar excreted in the urine despite normal levels in the blood) is suspected, urine samples may also be collected for testing along with the fasting and 2 hour blood tests.

The inventors have realized that a disadvantage of the OGTT is that the test is not representative for the physical response of the body to food uptake in real-life. In the normal diet almost never pure glucose is consumed. Therefore, it does not give a good physiological relevant image of the metabolic response to dietetic glucose exposure of the body. Therefore, the use of the classic OGTT results in false positive responses, *i.e.* patients which in real life will hardly experience problems may score high in a OGTT, and based on that result start medication or other interventions. The problem was also presented by Meier et al. (Diabetes, Obesity and Metabolism, 11, 2009, 213-222) where an OGTT was compared to a mixed meal tolerance (MMT) test (using a large Continental breakfast) and it was found that absolute levels of glycaemia greatly differ between both conditions. However, no solution to the problem was provided. Hence, it was found that it would be desirable to provide a better alternative for a mixed meal challenge which mimics the metabolic response of the body. A further disadvantage of the OGTT is that it requires the person taking the test to consume large amounts of sugar water, which is often experienced as unpleasant. This unpleasant procedure may discourage people to take the test. In addition, high amounts of glucose impair the normal dietetic pattern, especially in subjects that are suspected of suffering from a glucose-intolerant condition.

### PRIOR ART

WO 2005/017532 (CEAPRO Inc) discloses a solid diagnostic test meal for monitoring postprandial glucose to screen insulin levels and/or diagnose disorders of carbohydrate metabolism, comprising a polysaccharide. None of the disclosed compositions is a liquid composition comprising at least 90 weight% of glucose units, the meal is a dry solid product, of which 100 gram is taken with 450 ml of water within 10 minutes.

EP 1 424 074 A1 relates to a nutritional composition for therapeutic use, wherein the blood sugar level is controlled, for instance, in patients suffering from diabetes, glucose intolerance, or for obesity prevention. A diagnostic use of the composition for determining a glucose-intolerant condition is not disclosed.

WO 97/02050 (VEXHO Healthcare Inc) discloses a solid diagnostic test meal for the same use as in WO 2005/017532, found to produce a more precise measurement of postprandial glucose and insulin responses compared to a liquid meal or liquid glucose beverage. The use of such a liquid meal is dissuaded.

### SUMMARY OF THE INVENTION

An object of the present invention is to provide a liquid nutritional composition for performing a standardized test for screening a mammal for a glucose-intolerant condition, wherein one or more levels selected from the group of: the level of glucose, the level of one or more triglycerides, and the level of one or more gastrointestinal incretin hormones, such as gastric inhibitory polypeptide (GIP) and glucagon-likepeptide-1 (GLP-1) are measured.

A further object of the present invention is to provide a specific diagnostic method for screening a mammal for a glucose-intolerant condition as defined above, wherein respectively levels of glucose, triglycerides, and gastrointestinal incretin hormones, such as gastric inhibitory polypeptide (GIP) and glucagon-likepeptide-1 (GLP-1) are measured.

A further object of the invention is to provide a specific kit suitable for screening a mammal for a glucose-intolerant condition as defined above.

A further object of the invention is to provide a kit suitable for screening nutritional compositions on their post-prandial glucose response in a mammal ; in this case the liquid nutritional composition according to the invention will be used as a standard reference nutrition to which the tested nutritional compositions will be compared.

A further object of the invention is to provide a liquid nutritional composition which may serve as a meal replacer.

A further object of the invention is to provide a liquid nutritional composition which is safe, well-tolerated and does produce results which are less false positive, and give reproducible results, compared to prior art OGTT-test products.

In a first aspect, the invention is directed to a liquid nutritional composition comprising protein, fat, digestible carbohydrate and dietary fibre, wherein the digestible carbohydrate comprises at least 90 weight% of glucose units, based on total digestible carbohydrate mass, for use in a diagnostic method for screening a mammal for a glucose-intolerant condition. The inventors have realized that by using such a liquid nutritional composition in a diagnostic method for screening a mammal for a glucose-intolerant condition, a more accurate physiological relevant image of the metabolic response of the body can be obtained.

The composition of the invention is a liquid. A liquid composition is advantageous in that it passes the stomach easily and in a reproducible way, thus ensuring rapid and reproducible absorption of the glucose comprised in the product. In a further preferred embodiment, the composition comprises an emulsion, preferably essentially free of lipid particles with a particle diameter of 100 of larger.

Furthermore, compared to a solid meal, such a liquid offers several advantages : it can be administered more easily in a short (< 5 minutes) and/or fixed time, contrary to a solid meal which needs to be chewed before it is ingested, generating a larger time to ingest and a larger variability among test persons or the same person tested on different times. This is especially true for people with chewing or swallowing problems; also, in a preferred embodiment, the liquid composition has a prolonged shelf life (at least 6 months) as the liquid may be pasteurized or sterilized, contrary to the prior art OGTT test products, which needs to be prepared on the spot.

Preferably, the mammal is a human.

In a second aspect, the present invention provides a liquid nutritional composition comprising protein, fat, digestible carbohydrate and dietary fibre, wherein the digestible carbohydrate comprises at least 90 weight% of glucose units. The glucose units in a composition of the invention are readily absorbable into the blood. Said liquid nutritional composition is preferably at least substantially free of fructose.

In a third aspect, the present invention provides a diagnostic method for screening a mammal for a glucose-intolerant condition, comprising the step of administering the liquid nutritional composition of the invention. Due to the presence of nutrients in the composition besides glucose, a diagnostic method according to the invention is also called a meal tolerance test (MMT) or mixed-meal tolerance test (MMTT).

In a fourth aspect, the invention is directed to a kit for screening a mammal for a glucose-intolerant condition, comprising a liquid nutritional composition comprising protein, fat, digestible carbohydrate and dietary fibre, which digestible carbohydrate comprises at least 90 weight% of glucose units, a glucose level tester, and optionally, instructions for use.

In yet a further aspect, the reproducible glucose response allows comparison of responses between various meals, which may have a different composition or form.

According to a further embodiment, the composition according to the invention may also be used to detect and monitor a postprandial lipid, insulin, and/or incretin response.

### DETAILED DESCRIPTION OF THE INVENTION

In a first aspect, the invention is directed to a liquid nutritional composition comprising protein, fat, digestible carbohydrate and dietary fibre, wherein the digestible carbohydrate comprises at least 90 weight% of glucose units (based on the total digestible carbohydrate mass in the nutritional composition), (suitable) for use in a diagnostic method for screening a mammal for a glucose-intolerant condition.

The inventors surprisingly found that by using such a liquid nutritional composition in a diagnostic method for screening a mammal for a glucose-intolerant condition, an accurate physiological relevant image of the metabolic response of the body can be obtained. The inventors realized that the accurateness of the physiological relevant image of the metabolic response of the body can be improved by developing a test that resembles the uptake of glucose in a regular diet in everyday life as closely as possible (comparable to a mixed meal challenge). They further realized that carbohydrates are rarely consumed alone in a normal diet, but are in most cases consumed together with other macronutrients in a normal diet, such as protein, fat and dietary fibre.

The term "glucose-intolerant condition" as used in the present application is a condition wherein the glycaemic levels in a person are above 'normal'. The term is meant to comprise pre-diabetic conditions and/or diabetic conditions, as well as periods wherein glucose is not tolerated well due to circumstances or diseases, either temporarily or chronically. Examples of such conditions include conditions associated with persons experiencing insulin resistance, such as during aging, after surgery or during end-stage diseases and women suffering from gestational diabetes. Diabetes is defined herein as a condition wherein a person has a fasting glucose level of minimum 7.0 mmol/l. The term "glucose fasting level" as used herein refers to the glucose level in the blood of a person after having fasted for a predefined period of 10-14 hours. Pre-diabetes is defined herein as a condition wherein a person has a fasting glucose level above 6.0 mmol/l and below 7.0 mmol/l. Pre-diabetes may also be referred to as Intermediate Hyperglycaemia and includes Impaired Fasting Glucose (IFG) and Impaired Glucose Tolerance (IGT). IFG is defined herein as a condition wherein a person has a fasting glucose level of 6.1-6.9 mmol/l, as determined by the OGTT. IGT is defined herein as a condition wherein a person has a fasting glucose level below 7 mmol/l and a 2 hour glucose level of between 7.8 and 11.1 mmol/l, as determined by the OGTT. The term "2 hour glucose level" as used herein refers to the glucose level in the blood of a person measured two hours after consuming a certain amount of glucose, wherein the person has fasted for at least 6 hours prior to consumption.

The term "glucose unit" as used herein may be substituted at at least one of its hydroxyl groups, typically by esterification. Thus, glucose units form the constituents of glucose molecules (dextrose), oligosaccharides and polysaccharides. For example, the disaccharide maltose comprises two glucose units, while the maltodextrine molecule comprises multiple glucose units. It is apparent that the glucose units should be suitable for uptake in the blood of a subject. Generally, the glucose units are D-glucose units.

The term "fructose unit" as used herein may be substituted at at least one of its hydroxyl groups, typically by esterification. Thus, fructose units form the constituents of fructose molecules (fructose), oligosaccharides and polysaccharides. For example, the disaccharides sucrose and palatinose comprises one fructose unit (and one glucose unit). It is apparent that the fructose units should be suitable for uptake in the blood of a subject.

The terms "carbohydrate" and "digestible carbohydrate" are used interchangeably in this application. The terms "digestible carbohydrate" and "carbohydrate" as used herein refers to carbohydrate that is ordinarily digestible in the environment of the small intestines. A digestible carbohydrate can be a monosaccharide, an oligosaccharide or a polysaccharide. Dietary fibre (which may comprise indigestible oligosaccharide and/or indigestible polysaccharide) on the other hand is not a digestible carbohydrate.

As used herein, digestible carbohydrates are in particular carbohydrates which are released for more than 10 % of the sugars within 120 minutes in an analysis setting using standard digestive enzymes, as determinable by the Enquist method. Digestible carbohydrates can be divided into slowly digestible carbohydrates and rapidly digestible carbohydrates. Carbohydrates that are digested rapidly include maltodextrose, maltose or glucose (quickly digestible carbohydrates), and carbohydrates digested at least as quickly as maltodextrose, maltose or glucose. In particular, within 20 minutes, more than 90 % of quickly carbohydrates are digested in accordance with the Enquist method.

Preferably, 50 to 100 weight% of the glucose units, more preferably 90 to 100 weight% of the glucose units, in particular 95 to 100 weight%, more in particular 99 to 100 weight% of the glucose units in the composition is monosaccharidic glucose, in particular monosaccharidic D-glucose (dextrose, 'grape sugar', (2*R*,3*S*,4*R*,5*R*)-2,3,5,4,6-pentahydroxyhexanal). This has been found advantageous with respect to measuring a change in a relevant parameter in response to administration of a composition according to the invention in a diagnostic use according to the invention (such as glucose level, the triglyceride level, the gastrointestinal incretin hormones level, the insulin level, the lactate level, pH, the bicarbonate level, ketone bodies, carbon dioxide or any combination thereof).

Slowly digestible carbohydrates are digestible but less fast than maltodextrose, maltose and glucose. In particular, a carbohydrate is considered slowly digestible in case more than 10 % of the sugars is released within 20 and 120 minutes in an analysis setting using standard digestive enzymes, as determinable by the Enquist method.

Preferably, at least part of the digestible carbohydrate in a composition according to the invention comprises one or more quickly digestible carbohydrates. For example, resistant starch, which also comprises glucose units - may be present in a composition according to the invention, but if present, preferably only provides part of the digestible carbohydrate.

The term "about" is in particular used herein to indicate a range of ± 10 %, more in particular of ± 5 % around a given value.

The screening for a glucose-intolerant condition may comprise testing the mammal for one or more of the glucose-intolerant conditions from the group consisting of diabetes, gestational diabetes, pre-diabetes, impaired glucose tolerance, impaired fasting glycaemia, insulin resistance and reactive hypoglycaemia.

The screening usually comprises measuring one or more markers for a glucose-intolerant condition. In particular, levels of one or more compounds in blood, ocular fluid, urine, breath or any another bodily fluid or gas may be determined. When referring herein to 'blood' in relation to measuring a marker, the term 'blood' is meant to include whole blood, blood plasma and blood serum.

The screening may comprise measuring the glucose response.

The screening may comprise measuring the gastrointestinal incretin hormones response, such as gastric inhibitory polypeptide (GIP) and glucagon-like peptide-1 (GLP-1).

The measurements of the concentrations of glucose and incretin hormones can be performed in a manner commonly known in the art, *e.g.* as described in handbooks for clinical chemistry.

The screening may further comprise measuring the triglyceride response. The invention is particularly suitable for measuring the triglyceride response. The presence of fat in the liquid nutritional composition according to the invention enables the measuring of the triglyceride response of the body to ingested fats. This measurement is expected to be representative for the triglyceride response of the body after consuming a meal. Such measurements are not possible by using previous tolerance tests, such as OGTT.

Triglyceride response can be measured by a diagnostic method known in the art, *e.g.* as described in Clinical Chemistry, Vol. 41, No 10, 1995, pp. 1421-1426. Further, triglyceride response may be measured by the Centers for Disease Control and Prevention (CDC) triglyceride method.

The screening may further comprise the measurement of one or more of lactate, pH, bicarbonate and ketone bodies in blood. In particular, a decrease in lactate or ketone bodies or an increase of pH in blood are relevant. In breath samples, carbon dioxide concentrations can be determined.

The measurements of the concentrations of lactate, pH, and ketone bodies in body fluids and of carbon dioxide in breath can be performed in a manner commonly known in the art, *e.g.* as described in handbooks for clinical chemistry.

Preferably, the digestible carbohydrate comprises at least 95 weight%, more preferably 99 weight%, most preferably 99.9 weight% of glucose units, based on the total digestible carbohydrate mass in the liquid nutritional composition according to the invention. Glucose units are preferred over non-glucose units, because the body is not able to fully convert non-glucose units into glucose. Only glucose levels in the blood are measured in the tolerance test of the invention. Consequently, non-glucose units will not be effectively measured by the test, which measures glucose levels in the blood. For example, the monosaccharide galactose is only partly metabolized to glucose. Glucose levels measured, in the blood are therefore not fully representative for the amount of ingested sugar. In fact, ingested galactose will only show a glucose response that is about 23 % of the glucose response corresponding to an equal amount of ingested glucose.

The digestible carbohydrate may be chosen from glucose; dimers of glucose, such as maltose; oligomers of glucose; and polysaccharides such as maltodextrine, starch and glycogen, including any combination thereof.

Preferably, a starch fraction comprises less than 30 weight%, more preferably less than 22 weight% of "resistant" starch. Resistant starch demonstrates a slow release pattern in the gut and can be defined as those starches which in the digestion model as described later in this document, release more than 30% of their glucose units after 2 hours of digestion.

Preferably, the composition according to the invention comprises at least one carbohydrate chosen from the group of glucose, maltose, maltodextrine and starch.

In a specific embodiment, the liquid nutritional composition is at least substantially free of fructose. It was found that the presence of fructose in the composition influences the intake of glucose in the cells of the mammal, which may lead to errors in measuring glucose levels. With 'at least substantially free of fructose' is therefore in particularly meant that the fructose - if present at all - is present in the composition in a concentration that does not result in a significant adverse effect on the measured glucose level. Therefore, in this embodiment the carbohydrate usually comprises less than 3 weight%, preferably less than 1.0 weight%, more preferably less than 0.1 weight% of fructose, based on the total carbohydrate mass in the nutritional composition. The presence of a combination of dietary fibre, protein and fat in the composition is in particular advantageous, because thus a nutritional composition is obtained that closely resembles a regular meal consumed in real-life, which usually also contains such combination. Consequently, the liquid nutritional composition preferably comprises at least 10 weight% of fat, based on the total dry weight of the nutritional composition. Further, said liquid nutritional composition preferably comprises at least 1 weight%, more preferably at least 3 weight% of dietary fibre, based on the total dry weight of the nutritional composition.

Usually, the liquid nutritional composition comprises 40 to 80 weight%, preferably 50 to 70 weight% of digestible carbohydrate, based on the total dry weight of the nutritional composition. Typically, the liquid nutritional composition comprises 10 to 30 weight%, preferably 15 to 25 weight% of protein, based on the total dry weight of the nutritional composition. Proteins may play an important role in glucose consumption in the body of the mammal due to improved insulin secretion that may arise because of protein consumption.

As a protein source for the proteins in the composition, one or more compounds may be selected from the group of proteins derived from products having an animal origin, like dairy proteins, in particular whey, casein, caseinate, proteins from seeds or beans like soy and pea and cereal proteins like those derived from wheat or corn. Said protein source or part thereof may have been modified, in particular by (partial) hydrolysis.

With whey is meant a source of globular proteins that can be isolated from dairy products. In particular, globular whey proteins can be selected from beta-lactoglobulin, alpha-lactalbumin and serum albumin, including mixtures thereof. Examples of mixtures that comprise whey proteins are whey isolate and whey concentrate.

In a specific embodiment, it is preferred to use a combination of proteins which originate from vegetable and animal sources, in order to represent real-life situations. It is therefore preferred to include in this embodiment vegetable proteins and animal proteins in a weight ratio of 1: 5 to 5: 1.

Typically, the liquid nutritional composition comprises 10 to 30 weight%, preferably 15 to 25 weight% of fat, based on the total dry weight of the nutritional composition. Fat present in the composition may cause a delayed release of glucose in the gastrointestinal tract.

The fat in the composition may be chosen from one or more compounds from the group consisting of fatty acids, fatty acid derivatives (including tri-, di-, and monoglycerides, lyso-lecithins, glycolipids and phospholipids, sterol-containing metabolites such as cholesterol, and polyunsaturated fatty acids, such as ω-3 or ω-6 polyunsaturated fatty acids. However, in order to represent daily life situations, in one embodiment it is preferred that the lipid fraction comprises less than 1.5 weight% of (added) glycolipids and/or less than 10 weight% of mono and diglycerides. In a further embodiment the lipid fraction comprises more than 1 weight% of cholesterol or derivatives thereof, such as esters, to reflect a real-life diet. In a further embodiment, the lipid fraction comprises more than 13 g saturated fatty acids per 100 g fatty acids in the products, in order to reflect a real-life diet.

Typically, the liquid nutritional composition comprises 1 to 8 weight%, preferably 2 to 6 weight% of dietary fibre, based on the total dry weight of the nutritional composition. Dietary fibre may be selected from non-starch polysaccharides such as cellulose and many other plant components such as dextrins, inulin, lignin, waxes, chitins, pectins, beta-glucans and oligosaccharides.

Dietary fibre can be soluble (able to dissolve in water) or insoluble (not able to dissolve in water). Soluble fibre, like all fibre, cannot be digested by the human digestive enzymes. However, it can interact with receptors on the jejunal or intestinal epithelia or be fermented during its passage through the digestive tract, thus being wholly or partially transformed by gut bacteria into components which can be absorbed by the gut. The composition according to the invention suitable for use in the new glucose tolerance test preferably includes a fibre fraction which demonstrates an effect on the receptors in the jejunum and ileum, in particular the Toll-like receptors and/or lectin receptors.

Food sources of dietary fibre are often divided according to whether they provide (predominantly) soluble or insoluble fibre. To be precise, in general, both types of fibre are present in all plant foods, with varying degrees of each according to a plant's characteristics.

Potential advantages of consuming fibre are the production of health-promoting compounds during the fermentation of soluble fibre, and insoluble fibre's ability (via its passive water-attracting properties) to increase bulk, soften stool and shorten transit time through the intestinal tract.

Preferably, the liquid nutritional composition according to the invention comprises
40 to 80 weight%, more preferably 50 to 70 weight% of digestible carbohydrate;
10 to 30 weight%, more preferably 15 to 25 weight% of protein;
10 to 30 weight%, more preferably 15 to 25 weight% of fat;
1 to 8 weight%, more preferably 2 to 6 weight% of dietary fibre; based on the total dry weight of the nutritional composition.

The liquid nutritional composition preferably provides a dose of 1.0 to 2.5 g of glucose units, preferably 1.5 to 2.0 g of glucose units, per kilogram body weight of the mammal preferably up to a maximum dose of 75 g, when used for screening purposes. Such dosage makes the composition particularly suitable for the screening for glucose-intolerant conditions.

An additional advantage of the liquid nutritional composition according to the invention, is that a mammal consuming the composition has immediately obtained a complete nutritional meal. This is desirable, since glucose tolerance tests in which the composition may be used often require patients to arrive after fasting a number of hours. By consuming the liquid nutritional composition according to the invention, the patient will have satisfied his appetite at least in part and subsequently feel more comfortable during the test. Preferably, the liquid nutritional composition is a complete nutritional composition, which means that it comprises dietary fibre, protein and fat.

The nutritional composition according to the invention is a liquid composition, *e.g*. a drink. Preferably, the liquid nutritional composition according to the invention is heat-treated, i.e. it has undergone a pasteurisation and/or sterilisation treatment. By having subjected the liquid nutritional composition according to the invention to a heat-treatment, said composition has a long shelve-life, typically 6 months to 2 years.

Within the context of the invention, in general the shelve life of a product is the period, starting from its manufacture, during which the product remains suitable for consumption. In particular, during its shelf-life, the product maintains an acceptable microbiological quality and maintains fluidity. In a preferred embodiment, the product maintains a viscosity of about 200 mPa.s or less, more preferably of 100 mPa.s or less during its shelve life. Within the context of the invention, the viscosity is the viscosity as measurable using an Anton Paar Physica MCR301 rheometer with a CP50-1/PC cone (diameter 50 mm, 1° difference between middle and outside) at 20 0C at 100 s-1.

A sterilized or pasteurized liquid physical state is preferred over a sterilized or pasteurized solid product. It is contemplated that a sterilized/pasteurized liquid product with certain microbiological properties (such as a specific germ count) is provided under milder conditions than a comparable solid product, whereby undesired side-effects may be avoided or less prominent. Examples of such side-effects can include reduced organoleptic properties or shelve-life due to other factors than microbiological factors.

Within the context of the present invention, the term " sterilization treatment" and the term "sterilization" is meant to comprise any method using heat (sterilization, pasteurization), radiation (UV-treatment), and filtration (ultrafiltration, diafiltration, nanofiltration) to reduce the number of or remove possible pathogens. Preferably, the sterilization treatment includes a heat treatment at a high temperature for a short period, such as a UHT (Ultra High Temperature) treatment. Hence, within the context of the present invention, pasteurization is comprised within sterilization.

Within the context of the present invention, a "sterilized composition" is a composition that is obtained or obtainable by subjecting a composition to a sterilization treatment. In general, the quantity of potentially pathogenic micro-organisms of the sterilized composition meets food safety requirements, as applicable *e.g*. in the US or EU. In particular, a sterilized composition in accordance with the invention maintains to meet such requirement, for at least 6 months, preferably at least 12 months, when stored in a sealed packaging at ambient temperature (20 °C).

Preferably, the liquid nutritional composition according to the invention is emulsified in order to achieve a liquid homogeneous composition. This composition demonstrates a predictable behaviour regarding transit time through the stomach. This is desirable in order to secure a defined exposure of the human body to glucose via the oral route. The emulsified composition also demonstrates a predictable digestion pattern. This is desirable for the release of the digestible carbohydrates and lipid-derived and proteinaceous components from the product in the gut lumen and the activation of receptors which induce release of hormones, like insulin, glucagon and incretins and for activation of the neural system in the gut. These hormones and the neural system have a profound action on postprandial glucose profiles.

In a preferred embodiment, the liquid emulsion is essentially free of large lipid particles. In particular less than 5 volume% of the lipids is present in the form of particles which have a diameter larger than 100 micrometer. In a preferred embodiment, the liquid emulsion is also characterized by its way of manufacture. Preferably, at least one emulsification process is applied which uses a pressure above 500 bar. Preferably, the emulsification process is applied in at least two steps, one being applied before and one after a heating step of a liquid emulsion, which comprises at least part of the protein- and lipid fractions of the liquid nutritional composition according to the invention.

In a second aspect, the present invention provides a liquid nutritional composition comprising protein, fat, carbohydrate and dietary fibre, wherein the digestible carbohydrate comprises at least 90 weight% of glucose units, based on the total digestible carbohydrate mass in the nutritional composition, which is preferably substantially free of fructose. This liquid nutritional composition may have the same composition as the liquid nutritional composition of the first aspect.

In a third aspect, the composition of the invention is a liquid. A liquid composition is advantageous in that it passes the stomach easily and in a reproducible way, thus ensuring rapid and reproducible absorption of the glucose comprised in the product. In a further preferred embodiment, the composition comprises an emulsion, preferably essentially free of lipid particles with a particle diameter of 100 µm of larger.

In a fourth aspect, the present invention provides a diagnostic method for screening a mammal for a glucose-intolerant condition, comprising the step of administering the liquid nutritional composition of the invention to the mammal, the diagnostic method further comprising taking at least one sample of a bodily fluid - typically at least one of blood, saliva, expired breath, ocular fluid and urine - prior to administration, taking at least one sample of the bodily fluid after administration, and determining the concentration of at least one analyte, such as at least one analyte selected from the group of glucose, triglycerides, gastrointestinal incretin hormones and insulin, in said samples. Such a diagnostic method was found to give a good physiological relevant image of the metabolic response of the mammal's body. In particular it provides useful information about glucose tolerance in the real life situation. More in particular, it addresses inter-individual differences in digestion patterns of different food components and inter-individual differences in response pattern of incretin hormone and neuronal responses resulting from the intake of a mixed-component meal.

As used herein, a physiological relevant image at least includes the glucose response, as determined with a diagnostic method according to the invention. In a special embodiment, the glucose response according the present invention is characterized by specific parameters, which are derived from the concentration measurements. Further, the image may include at least one of the following: the triglyceride response, gastrointestinal incretin hormone response and insulin response.

In a fifth aspect, the invention is directed to a kit for screening a mammal for a glucose-intolerant condition, comprising a liquid nutritional composition comprising protein, fat, digestible carbohydrate and dietary fibre, which digestible carbohydrate comprises at least 90 weight% of glucose units, a glucose level tester, and optionally, instructions for use.

In a sixth aspect, the present invention provides a diagnostic method for screening nutritional compositions on their post-prandial glucose response, in this case the liquid nutritional composition according to the invention will be used as a standard reference nutrition, to which all nutritional compositions tested will be compared.

The advantage of using the MMT over the OGTT for screening of glucose intolerant conditions is that an MMT gives a better physiological relevant image of the metabolic response of the body. The MMT may be used for testing a mammal for one or more of the glucose-intolerant conditions from the group consisting of diabetes, gestational diabetes, pre-diabetes, impaired glucose tolerance, impaired fasting glycaemia, insulin resistance and reactive hypoglycaemia. The MMT may be also be used as an alternative for the oral glucose tolerance test (OGTT). A further advantage of using an MTT over an OGTT is that the MTT is more suited for use in malnourished individuals. Chronically-ill patients and elderly can become diseased or age induced fragile. With an MTT, the fasting period is reduced compared to an OGTT. An other advantage is compliance to the protocol. Patients will tend to violate the protocol subscription (no oral intake of nutritional products, or sugar containing drinks, other than those subscribed by the protocol) if they experience less hunger feelings. An advantage of the MMT according to the invention over the common MMT is the fact that the test product can be standardized, and hence, test results are comparable.

Accordingly, in the diagnostic method of the invention the liquid nutritional composition of the invention is preferably administered to a mammal in a dose of 1.0 to 2.5 g of glucose, more preferably 1.5 to 2.0 g of glucose, per kilogram body weight of the mammal. In practice, if the mammal is a human, a composition comprising about 75 grams of digestible carbohydrates may suitably be administered according to the diagnostic method of the invention. However, the exact dose may depend on the weight of the mammal. For example, a full adult dose of 75 g should not be given to a person weighing less than 43 kg, because exaggerated glucoses may in such a case produce a false positive result.

The diagnostic method of the invention may further comprise the step of measuring the glucose level in the mammal. Said measurement may be performed on blood drawn from the patient, or alternatively, in an ocular fluid of the patient as disclosed in US 2007/0154395.

The patient is instructed not to restrict carbohydrate intake in the days or weeks before the MMT method according to the invention. He should preferably eat a normal, balanced diet the week before taking the test. A diet including at least 150 to 200 grams of carbohydrates per day is recommended. The MMT method according to the invention is preferably done on a mammal in good health, as results from a mammal during an illness may not reflect the mammal's glucose metabolism when healthy.

Preferably, the mammal has been fasting for 8 to 14 hours prior to administering. While fasting, the mammal may drink water, but should abstain from nutritional foods in either solid or liquid form, for example nutritional foods comprising carbohydrate, fat and proteins.

Preferably, the diagnostic method according to the invention is conducted in the morning, *e.g*. between 7 and 8 am, as glucose tolerance exhibits a diurnal rhythm with a significant decrease in the afternoon.

Preferably, the composition is administered over a period of time less than 10 minutes, preferably less than 5 minutes. The composition may be provided in any form, *e.g.* a solid or liquid form. Preferably, the composition is provided in the form of a drink.

Preferably, the diagnostic method also comprises the step of data analysis. It appeared to the inventors that several characteristics of the analyte-time profile have to be simultaneously taken into account in order to draw the proper conclusions about meal tolerance. Considering for example the glucose-time profile as measured in blood after the meal challenge, it appears important to determine at least 2 and preferably 3 and more preferably 4 of the following parameters:
1- Time to reach the maximum value (peak value) ;
2- Magnitude of the peak value ;
3- Clearance time, as reflected in the halftime value t^{1/2}, i.e. the time to have the concentration of analyte in the samples decrease to half the original value ;
4- Baseline value, as measured by the value of the analyte briefly (1 to 30 minutes) before the administration of the product, and the equilibrium value after complete digestion and absorption of the meal according the invention ;
5- Shape and symmetry of the profile ;
6- Incremental area under the curve (IAUC) as measured between 2 time points which are selected briefly before the start of the digestion and briefly after the complete digestion of the product. Preferably for the product according the invention this occurs between 5 and 180 minutes after administration of the meal.
7- IAUC between 2 times points, which are selected to be 20 minutes before the peak time (after part of the product has already been absorbed) and one halftime value after the peak time of the analyte in the samples. For the product according the invention, these time points are typically 15 and 120 minutes after administration of the meal.

It appeared that a useful parameter is the ratio of parameter 2 to parameter 1. The higher the ratio is, the higher the peak value and the shorter the time to reach it, and the better the absorption and/or the slower the capacity of the body to adapt to the meal challenge. It appeared to the inventors that the value of this ratio is an independent predictor of the risk of developing diabetes, obesitas and metabolic syndrome.

In order to obtain a reliable insight in the long term effects of the body to a meal, it appeared useful to use the value of the multiplication of clearance time (parameter 3) and baseline (parameter 4). This number reflects the capacity of the body to remove absorbed glucose or lipid. The higher the number, the more problems the body will experience on the longer term. This number reflects therefore the risk on developing problems with vision (e.g. cataract), vascular problems, hypertension and kidney problems. The number can optionally be used in combination with the values for the concentration of glycosylated hemoglobine (HbA1C) or advanced glycation products.

The values of parameters 6 and 7, and in particular their ratio, reflect in an alternative way the information as obtained by using the ratio of parameters 2 to 1.

For the interpretation of the determined values for triglycerides, similar parameters can be applied. For the interpretation of the determined values for lactate and ketone bodies, especially the degree and rate of decrease in concentration compared to baseline values, are of interest.

The diagnostic method according to the invention for screening a mammal for a glucose-intolerant condition may comprise the following subsequent steps of:
- taking a first sample from said mammal ; this sample can be taken from any bodily fluid or gas, as defined above ;
- administering the mammal the liquid nutritional composition according to the invention ;
- taking a second sample from said mammal after administering said liquid nutritional composition;
- measuring a parameter in said first and/or second sample;
- comparing the measured level of said parameter or combinations thereof with standard values or a desirable value.

The diagnostic method may further comprise the step of making a diagnosis based on the data obtained by measuring and comparing the levels of one or more parameters.

In particular, the diagnostic method according to the invention for screening a mammal for a glucose-intolerant condition may comprise the steps of:
- taking a first sample from said mammal that has preferably been fasting for 8 to 14 hours; followed by
- administering to the mammal the liquid nutritional composition according to the invention, wherein the composition is preferably administered orally and over a period of time less than 5 minutes; followed by
- taking a second sample from said mammal after administering said liquid nutritional composition, preferably about 0.25 to 4 hours, more preferably about 0.5 to 2 hours after consumption of said liquid nutritional composition,
- measuring the glucose level of said first and second sample;
- comparing the measured glucose levels with standard values, or a desirable value, preferably adapted for the mammal under investigation, such as for age, gender, etc..In a healthy human subject, the 2-hour glucose level measured with the OGTT test of the prior art is typically below 7.8 mmol/l (140 mg/dl). Glucose levels between 7.8 and 11.1 mmol/l (200 mg/dl) indicate "impaired glucose tolerance." Glucose levels above 11.1 mmol/l (200 mg/dl) at 2 hours confirms a diagnosis of diabetes. The ingestion of the liquid nutritional composition of the invention may result in a milder response of the body compared to the OGTT test, *viz.* glucose values are measured in MMT tests after ingestion of the composition that are 50 to 90% of the above values regarding the OGTT test, probably about 75%. Such a mild response is thought to be advantageous regarding the health of the subject taking the test.

If desired, more than two samples are taken. Samples may be taken at intervals for measurement of glucose (blood sugar), and sometimes insulin levels, which may also be determined in a manner commonly known in the art using antibodies, as described in Clinical Handbooks, such as a double antibody immune assay, a radio immune assay and an immunochemiluminescence assay. The intervals and number of samples vary according to the purpose of the test. In research settings, samples may be taken on many different time schedules.

The glucose level measured in the first sample is called the fasting glucose level. This measurement may function as a baseline with which the glucose levels of the one or more other samples can be compared.

After fasting, the normal blood glucose rate is 60 to 100 mg/dL (milligrams per deciliter), preferably below 6.1 mmol/l (110 mg/dl). Fasting glucose levels between 6.1 and 7.0 mmol/l (110 and 126 mg/dl) are borderline ("impaired fasting glycaemia"), and fasting glucose levels repeatedly at or above 7.0 mmol/l (126 mg/dl) are diagnostic of diabetes. These fasting levels are determined after fasting and prior to administration of any nutritional composition. These levels may also be referred to as the "0 hour sample", for example in experiments.

The diagnostic method of the invention may further comprise the step of taking urine samples. Such samples may indicate renal glycosuria, *i.e.* sugar excreted in the urine despite normal levels in the blood.

The MMT method of the invention may *inter alia* reveal how quickly glucose is metabolized from the bloodstream for use by cells as an energy source. The normal rate of glucose clearing depends on the amount of glucose ingested.

A standard 2-hour MMT is normally sufficient to diagnose or exclude all forms of diabetes mellitus at all but the earliest stages of development. Longer tests may be used for a variety of other purposes, such as detecting reactive hypoglycaemia or defining subsets of hypothalamic obesity. Insulin levels may be measured to detect insulin resistance or deficiency.

### EXAMPLES

### Example 1:

Table 1 shows an example of a liquid nutritional composition according to the invention.

**Table 1**

| | Amount /100 ml | | |
|---|---|---|---|
| **Energy** | **150** | **kcal** | |
| **Protein** | **6** | **g** | **16 En%** |
| Ca-caseinate/Na-caseinate | 5.9 | g | |
| Soy | 0.1 | g | |
| **Carbohydrates** | **18.75** | **g** | **50 En%** |
| glucose | 0.37 | g | |
| lactose | < 0.03 | g | |
| maltose | 1.3 | g | |
| maltodextrine | 16.7 | g | |
| others | 0.38 | g | |
| **Fat** | **5.7** | **g** | **34 En%** |
| **Fibres** | **1.5** | g | |

| **Minerals and trace elements** | | | |
|---|---|---|---|
| - Sodium | 134 | mg | |
| - Potassium | 201 | mg | |
| - Chloride | 167 | mg | |
| - Calcium | 108 | mg | |
| - Phosphorus | 108 | mg | |
| - Magnesium | 34 | mg | |
| Ca/Pratio | 1 | mg | |
| - Iron | 2.4 | mg | |
| - Zinc | 1.8 | mg | |
| - Copper | 270 | µg | |
| - Manganese | 0.5 | mg | |
| - Fluoride | 0.2 | mg | |
| - Molybdenum | 15 | µg | |
| - Selenium | 8.6 | µg | |
| - Chromium | 10 | µg | |
| - Iodine | 20 | µg | |

| **Vitamins** | | | |
|---|---|---|---|
| - Vitamin A | 123 | µg-RE | |
| - Vitamin D3 | 1.1 | µg | |
| - Vitamin E | 1.9 | mg-a-TE | |
| - Vitamin K | 8 | µg | |
| - Thiamin (B1) | 0.2 | mg | |
| - Riboflavin (B2) | 0.2 | mg | |
| - Niacin (B3) | 2.7 | mg-NE | |
| - Pantothenic acid (B5) | 0.8 | mg | |
| - Vitamin B6 | 0.3 | mg | |
| - Folic acid | 40 | µg | |
| - Vitamin B12 | 0.3 | µg | |
| - Biotin | 6 | µg | |
| - Vitamin C | 15 | mg | |

### Example 2:

The glucose response of a composition according to the invention comprising 25 g digestible carbohydrate ("MTT 1.5") was compared to the glucose response of a glucose composition comprising 25 g glucose dissolved in water ("Glucose").

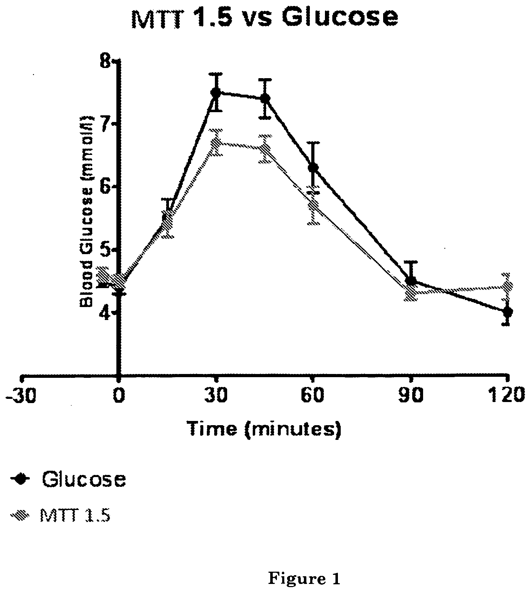

First, the effect on glucose levels in the blood was measured on a person who ingested the glucose composition after having fasted for at least 8 hours. A few days later, the effect on glucose levels in the blood was measured on the same person who ingested the digestible carbohydrate composition after having fasted for at least 8 hours. The results can be seen in Figure 1 and is in line with the findings by Meier et al (Diabetes, Obesity and Metabolism, 11, 2009, 213-222).

### Example 3:

Nutritional product for oral intake, being a liquid emulsion. The emulsion has a small average particle size below 10 micrometer and the amount of lipid particles having a diameter larger than 100 micrometer is less than 3 vol%. The ingredients as used during manufacture are (per 100 ml):

| | |
|---|---|
| Dairy proteins: | 4 g |
| Vegetable proteins: | 3 g |
| Digestible carbohydrates | 17 g, being maltodextrines |
| Lipids | 6 g |
| Fiber | 1 g |

Mineral and vitamin premixes to comply with the recommendations for food dor special medical purposes (FSMP).

## Claims

1. A liquid nutritional composition for use in a diagnostic method for screening a mammal for a glucose-intolerant condition, wherein the glucose-intolerant condition is selected from the group of pre-diabetic conditions and diabetic conditions, the composition comprising, based on the total dry weight of the nutritional composition, 10 to 30 weight % protein, 10 to 30 weight% fat, 40 to 80 weight% digestible carbohydrate and 1 to 8 weight% dietary fibre, wherein the digestible carbohydrate comprises at least 90 weight% of glucose units, based on total digestible carbohydrate mass.

2. Liquid nutritional composition according to claim 1, wherein the screening comprises measuring the glucose level, the triglyceride level, the gastrointestinal incretin hormones level, the insulin level, the lactate level, pH, the bicarbonate level, ketone bodies, carbon dioxide or any combination thereof, the screening preferably comprising measuring one or more of the glucose level, the triglyceride level and the gastrointestinal incretin hormones level.

3. Liquid nutritional composition according to any one of the previous claims, wherein the digestible carbohydrate is chosen from the group of glucose, maltose, maltodextrine, starch, glycogen and any combination thereof.

4. Liquid nutritional composition according to any one of the previous claims, wherein the nutritional composition comprises a dose of 1.0 to 2.5 g of glucose units, per kilogram body weight of the mammal, preferably up to a maximum of 75 g of glucose units.

5. Liquid nutritional composition according to any one of the previous claims, wherein the nutritional composition comprises a dose of 1.5 to 2.0 g of glucose units, per kilogram body weight of the mammal, preferably up to a maximum of 75 g of glucose units.

6. A liquid nutritional composition comprising protein, fat, digestible carbohydrate and dietary fibre, wherein the digestible carbohydrate comprises at least 90 weight% of glucose units, based on the total digestible carbohydrate mass in the nutritional composition, and which is at least substantially free of fructose, the liquid nutritional composition preferably comprising
40 to 80 weight% of digestible carbohydrate;
10 to 30 weight% of protein;
10 to 30 weight% of fat;
1 to 8 weight of dietary fibre;
based on the total dry weight of the nutritional composition.

7. Liquid nutritional composition according to any of claims 1-6, wherein the composition is an emulsion, wherein preferably less than 5 volume% of the lipids is present in the form of particles which have a diameter larger than 100 micrometer.

8. Liquid nutritional composition according to any of claims 1 to 7, wherein the digestible carbohydrate comprises at least 95 weight%, preferably 99 weight%, even more preferably 99.9 weight% of glucose units, based on the total digestible carbohydrate mass in the nutritional composition.

9. Liquid nutritional composition according to any of claims 1 to 8, wherein the digestible carbohydrate comprises less than 3 weight%, preferably less than 1 weight%, more preferably less than 0.1 weight% of fructose, based on the total digestible carbohydrate mass in the nutritional composition.

10. Liquid nutritional composition according to any of claims 1 to 9, comprising at least 10 weight%, preferably at least 20 weight% of fat and at least 1 weight%, preferably at least 4 weight% of dietary fibre, based on the total dry weight of the nutritional composition.

11. Liquid nutritional composition according to any of claims 1 to 10, comprising
50 to 70 weight% of digestible carbohydrate;
15 to 25 weight% of protein;
15 to 25 weight% of fat;
2 to 6 weight% of dietary fibre;
based on the total dry weight of the nutritional composition.

12. Liquid nutritional composition according to any one of claims 1 to 11, wherein 50 to 100 weight% of the glucose units, preferably 90 to 100 weight% of the glucose units, in particular 95 to 100 weight%, more in particular 99 to 100 weight% of the glucose units in the composition is monosaccharidic glucose.

13. Use of a liquid nutritional composition according to any one of claims 1 to 12, for the manufacture of a composition for a diagnostic method for screening a mammal for a glucose-intolerant condition, comprising the subsequent steps of:
- taking a first sample from said mammal that has preferably been fasting for 8 to 14 hours ; followed by
- administering to the mammal the nutritional composition according to any one of claims 1 to 12, wherein the composition is preferably administered orally and over a period of time less than 5 minutes ; followed by
- taking a second sample from said mammal after administering said nutritional composition, preferably about 0.25 to 4 hours, more preferably about 0.5 to 2 hours after consumption of said nutritional composition;
- measuring a parameter, preferably the glucose level, of said first and second sample;
- comparing the measured levels of said parameter or combinations thereof with standard values or a desirable value.

14. Use according to claim 13, wherein the diagnostic method further comprises measuring the triglyceride level of the first and of the second sample.

15. Kit suitable for screening a mammal for a glucose-intolerant condition, which kit comprises a nutritional composition according to any one of claims 1 to 12, a glucose level tester, and optionally instructions for use.

## Patentansprüche

1. Flüssige Ernährungszusammensetzung zur Verwendung in einem Diagnoseverfahren für das Screenen eines Säugers auf einen Glukoseintoleranzzustand, wobei der Glukoseintoleranzzustand ausgewählt ist aus der Gruppe vordiabetischer Zustände und diabetischer Zustände, die Zusammensetzung umfassend, basierend auf dem Gesamttrockengewicht der Ernährungszusammensetzung, 10 bis 30 Gew.-% Protein, 10 bis 30 Gew.-% Fett, 40 bis 80 Gew.-% verdauliche Kohlehydrate und 1 bis 8 Gew.-% Ballaststoffe, wobei das verdauliche Kohlehydrat, basierend auf der Gesamtmasse an verdaulichem Kohlehydrat, mindestens 90 Gew.-% Glukoseeinheiten umfasst.

2. Flüssige Ernährungszusammensetzung nach Anspruch 1, wobei das Screenen das Messen des Glukosespiegels, des Triglyceridspiegels, des gastrointestinalen Inkretinhormonspiegels, des Insulinspiegels, des Lactatspiegels, des pH, des Bicarbonatspiegels, der Ketonkörper, des Kohlendioxids oder jeder Kombination davon umfasst, wobei das Screenen bevorzugt das Messen von einem oder mehr des Glukosespiegels, des Triglyceridspiegels und des gastrointestinalen Inkretinhormonspiegels umfasst.

3. Flüssige Ernährungszusammensetzung nach einem der vorhergehenden Ansprüche, wobei das verdauliche Kohlehydrat aus der Gruppe von Glukose, Maltose, Maltodextrin, Stärke, Glykogen und jeder Kombination davon ausgewählt ist.

4. Flüssige Ernährungszusammensetzung nach einem der vorhergehenden Ansprüche, wobei die Ernährungszusammensetzung eine Dosis von 1,0 bis 2,5 g Glukoseeinheiten pro Kilogramm Körpergewicht des Säugers, bevorzugt bis zu einem Maximum von 75 g Glukoseeinheiten, umfasst.

5. Flüssige Ernährungszusammensetzung nach einem der vorhergehenden Ansprüche, wobei die Ernährungszusammensetzung eine Dosis von 1,5 bis 2,0 g Glukoseeinheiten pro Kilogramm Körpergewicht des Säugers, bevorzugt bis zu einem Maximum von 75 g Glukoseeinheiten, umfasst.

6. Flüssige Ernährungszusammensetzung, umfassend Protein, Fett, verdauliches Kohlehydrat und Ballaststoffe, wobei das verdauliche Kohlehydrat mindestens 90 Gew.-% an Glukoseeinheiten, basierend auf der Gesamtmasse an verdaulichem Kohlehydrat in der Ernährungszusammensetzung, umfasst, und die zumindest im Wesentlichen frei von Fructose ist, die flüssige Ernährungszusammensetzung bevorzugt umfassend:
40 bis 80 Gew.-% verdauliche Kohlehydrate;
10 bis 30 Gew.-% Protein;
10 bis 30 Gew.-% Fett;
1 bis 8 Gew.-% Ballaststoffe;
basierend auf dem Gesamttrockengewicht der Ernährungszusammensetzung.

7. Flüssige Ernährungszusammensetzung nach einem der Ansprüche 1-6, wobei die Zusammensetzung eine Emulsion ist, wobei bevorzugt weniger als 5 Vol.-% der Lipide in Form von Teilchen mit einem Durchmesser größer als 100 Mikrometer anwesend ist.

8. Flüssige Ernährungszusammensetzung nach einem der Ansprüche 1 bis 7, wobei das verdauliche Kohlehydrat mindestens 95 Gew.-%, bevorzugt 99 Gew.-%, noch bevorzugter 99,9 Gew.-% Glukoseeinheiten, basierend auf der Gesamtmasse an verdaulichem Kohlehydrat in der Ernährungszusammensetzung, umfasst.

9. Flüssige Ernährungszusammensetzung nach einem der Ansprüche 1 bis 8, wobei das verdauliche Kohlehydrat, basierend auf der Gesamtmasse an verdaulichem Kohlehydrat in der Ernährungszusammensetzung, weniger als 3 Gew.-%, bevorzugt weniger als 1 Gew.-%, bevorzugter weniger als 0,1 Gew.-% Fructose umfasst.

10. Flüssige Ernährungszusammensetzung nach einem der Ansprüche 1 bis 9, umfassend mindestens 10 Gew.-%, bevorzugt mindestens 20 Gew.-% Fett und mindestens 1 Gew.-%, bevorzugt mindestens 4 Gew.-% Ballaststoffe, basierend auf dem Gesamttrockengewicht der Ernährungszusammensetzung.

11. Flüssige Ernährungszusammensetzung nach einem der Ansprüche 1 bis 10, umfassend
50 bis 70 Gew.-% verdauliches Kohlehydrat;
15 bis 25 Gew.-% Protein;
15 bis 25 Gew.-% Fett;
2 bis 6 Gew.-% Ballaststoffe;
basierend auf dem Gesamttrockengewicht der Ernährungszusammensetzung.

12. Flüssige Ernährungszusammensetzung nach einem der Ansprüche 1 bis 11, wobei 50 bis 100 Gew.-% der Glukoseeinheiten, bevorzugt 90 bis 100 Gew.-% der Glukoseeinheiten, insbesondere 95 bis 100 Gew.-%, ganz besonders 99 bis 100 Gew.-% der Glukoseeinheiten in der Zusammensetzung monosaccharidische Glukose ist.

13. Verwendung einer flüssigen Ernährungszusammensetzung nach einem der Ansprüche 1 bis 12 zur Herstellung einer Zusammensetzung für ein Diagnoseverfahren für das Screenen eines Säugers auf einen Glukoseintoleranzzustand, umfassend die nachfolgenden Schritte von:
- Entnehmen einer erste Probe vom Säuger, der bevorzugt während 8 bis 14 Stunden gefastet hat; gefolgt von
- Verabreichen der Ernährungszusammensetzung nach einem der Ansprüche 1 bis 12 an den Säuger, wobei die Zusammensetzung bevorzugt oral und über einen Zeitraum von weniger als 5 Minuten verabreicht wird; gefolgt von
- Entnehmen einer zweiten Probe vom Säuger nach dem Verabreichen der Ernährungszusammensetzung, bevorzugt ungefähr 0,25 bis 4 Stunden, bevorzugter ungefähr 0,5 bis 2 Stunden nach dem Konsum der Ernährungszusammensetzung;
- Messen eines Parameter, bevorzugt des Glukosespiegels, der ersten und zweiten Probe;
- Vergleichen der gemessenen Spiegel des Parameters oder Kombinationen davon mit Standardwerten oder einem gewünschten Wert.

14. Verwendung nach Anspruch 13, wobei das Diagnoseverfahren ferner das Messen des Triglyceridspiegels der ersten und der zweiten Probe umfasst.

15. Kit, geeignet zum Screenen eines Säugers auf einen Glukoseintoleranzzustand, wobei das Kit eine Ernährungszusammensetzung nach einem der Ansprüche 1 bis 12, einen Glukosespiegeltester und optional Gebrauchsanweisungen umfasst.

## Revendications

1. Composition nutritionnelle liquide destinée à être utilisée dans un procédé de diagnostic pour dépister un mammifère concernant une affection intolérante au glucose, où l'affection intolérante au glucose est choisie dans le groupe des affections pré-diabétiques et des affections diabétiques, la composition comprenant, sur la base du poids sec total de la composition nutritionnelle, 10 à 30 % en poids de protéine, 10 à 30 % en poids de graisse, 40 à 80 % en poids de glucide digestible et 1 à 8 % en poids de fibre alimentaire, où le glucide digestible comprend au moins 90 % en poids d'unités de glucose, sur la base de la masse totale de glucide digestible.

2. Composition nutritionnelle liquide selon la revendication 1, où le dépistage comprend la mesure de la concentration de glucose, de la concentration de triglycérides, de la concentration d'hormones incrétines gastro-intestinales, de la concentration d'insuline, de la concentration de lactate, du pH, de la concentration de bicarbonate, des corps cétoniques, du dioxyde de carbone ou de toute combinaison de ceux-ci, le dépistage comprenant de préférence la mesure d'une ou plusieurs de la concentration de glucose, de la concentration de triglycérides et de la concentration d'hormones incrétines gastro-intestinales.

3. Composition nutritionnelle liquide selon l'une quelconque des revendications précédentes, où le glucide digestible est choisi dans le groupe du glucose, du maltose, de la maltodextrine, de l'amidon, du glycogène et de toute combinaison de ceux-ci.

4. Composition nutritionnelle liquide selon l'une quelconque des revendications précédentes, où la composition nutritionnelle comprend une dose de 1,0 à 2,5 g d'unités de glucose, par kilogramme de poids corporel du mammifère, de préférence jusqu'à un maximum de 75 g d'unités de glucose.

5. Composition nutritionnelle liquide selon l'une quelconque des revendications précédentes, où la composition nutritionnelle comprend une dose de 1,5 à 2,0 g d'unités de glucose, par kilogramme de poids corporel du mammifère, de préférence jusqu'à un maximum de 75 g d'unités de glucose.

6. Composition nutritionnelle liquide comprenant une protéine, une graisse, un glucide digestible et une fibre alimentaire, où le glucide digestible comprend au moins 90 % en poids d'unités de glucose, sur la base de la masse totale de glucide digestible dans la composition nutritionnelle, et qui est dépourvue au moins sensiblement de fructose, la composition nutritionnelle liquide comprenant de préférence
40 à 80 % en poids de glucide digestible ;
10 à 30 % en poids de protéine ;
10 à 30 % en poids de graisse ;
1 à 8 % en poids de fibre alimentaire ;
sur la base du poids sec total de la composition nutritionnelle.

7. Composition nutritionnelle liquide selon l'une quelconque des revendications 1-6, où la composition est une émulsion, où de préférence moins de 5 % en volume des lipides sont présents sous forme de particules qui ont un diamètre supérieur à 100 micromètres.

8. Composition nutritionnelle liquide selon l'une quelconque des revendications 1 à 7, où le glucide digestible comprend au moins 95 % en poids, de préférence 99 % en poids, de préférence encore 99,9 % en poids d'unités de glucose, sur la base de la masse totale de glucide digestible dans la composition nutritionnelle.

9. Composition nutritionnelle liquide selon l'une quelconque des revendications 1 à 8, où le glucide digestible comprend moins de 3 % en poids, de préférence moins de 1 % en poids, de préférence encore moins de 0,1 % en poids de fructose, sur la base de la masse totale de glucide digestible dans la composition nutritionnelle.

10. Composition nutritionnelle liquide selon l'une quelconque des revendications 1 à 9, comprenant au moins 10 % en poids, de préférence au moins 20 % en poids de graisse et au moins 1 % en poids, de préférence au moins 4 % en poids de fibre alimentaire, sur la base du poids sec total de la composition nutritionnelle.

11. Composition nutritionnelle liquide selon l'une quelconque des revendications 1 à 10 comprenant
50 à 70 % en poids de glucide digestible ;
15 à 25 % en poids de protéine ;
15 à 25 % en poids de graisse ;
2 à 6 % en poids de fibre alimentaire ;
sur la base du poids sec total de la composition nutritionnelle.

12. Composition nutritionnelle liquide selon l'une quelconque des revendications 1 à 11, où 50 à 100 % en poids des unités de glucose, de préférence 90 à 100 % en poids des unités de glucose, en particulier 95 à 100 % en poids, plus particulièrement 99 à 100 % en poids des unités de glucose dans la composition sont du glucose monosaccharidique.

13. Utilisation d'une composition nutritionnelle liquide selon l'une quelconque des revendications 1 à 12 pour la fabrication d'une composition pour un procédé de diagnostic pour dépister un mammifère concernant une affection intolérante au glucose, comprenant les étapes subséquentes de :
- prélèvement d'un premier échantillon sur ledit mammifère qui a de préférence été à jeun pendant 8 à 14 heures ; puis
- administration au mammifère de la composition nutritionnelle selon l'une quelconque des revendications 1 à 12, où la composition est de préférence administrée oralement et sur une durée inférieure à 5 minutes, puis
- prélèvement d'un second échantillon sur ledit mammifère après l'administration de ladite composition nutritionnelle, de préférence environ 0,25 à 4 heures, de préférence encore environ 0,5 à 2 heures après la consommation de ladite composition nutritionnelle ;
- mesure d'un paramètre, de préférence la concentration de glucose, dudit premier et second échantillon ;
- comparaison des concentrations mesurées dudit paramètre ou de combinaisons de ceux-ci avec des valeurs standards ou une valeur souhaitable.

14. Utilisation selon la revendication 13, où le procédé de diagnostic comprend en outre la mesure de la concentration de triglycérides du premier et du second échantillon.

15. Kit approprié pour dépister un mammifère concernant une affection intolérante au glucose, lequel kit comprend une composition nutritionnelle selon l'une quelconque des revendications 1 à 12, un dispositif de test des concentrations de glucose et éventuellement des instructions pour l'utilisation.
